# EUROPEAN PATENT APPLICATION

(11) **EP 2 544 111 A1**
(43) Date of publication of application: **09.01.2013**
(21) Application number: 11172888.7
(22) Date of filing: 06.07.2011
(51) Int. Cl.: G06F 19/00

(54) **System for managing cardiovascular health status**

(71) Applicant: Koninklijke Philips Electronics N.V., 5621 BA Eindhoven (NL)
(72) Inventor: Dittmer, Wendy, 5600 AE Eindhoven (NL); Waanders, Leonie, F., 5600 AE Eindhoven (NL); Dhand, Harsh, 5600AE Eindhoven (NL); Bussa, Nagaraju, 5600 AE Eindhoven (NL); Alves De Inda, Marcia, 5600 AE Eindhoven (NL); Mani, Arun, K., 5600 AE Eindhoven (NL); Dittmer, Janke, J., 5600 AE Eindhoven (NL)
(74) Representative: Damen, Daniel Martijn

(57) **Abstract**

A system (1) for computing a disease risk score of a patient based on a recommendation of treatment or lifestyle changes is disclosed. The system comprises an input device (11) for receiving risk parameter values for computing the disease risk score according to a risk score algorithm, and for receiving the recommendation of treatment or lifestyle changes, an interpreter (21) for computing at least one risk parameter value for computing the disease risk score, on the basis of the recommended treatment or lifestyle changes, a risk calculator (31) for calculating the disease risk score on the basis of the received risk parameter values and the computed risk parameter value, and an output device (41) for communicating the calculated disease risk score. Thanks to the interpreter (21), the recommended treatment or lifestyle change is "translated" into a value of a risk parameter for computing the disease risk score, thereby allowing calculating the risk score resulting from the treatment or lifestyle change.

## Description

### FIELD OF THE INVENTION

The invention relates to planning treatment of a patient at risk or already suffering from a disease such as cardiovascular disease.

### BACKGROUND OF THE INVENTION

Cardiovascular disease (CVD) is one of the leading causes of death worldwide. Unlike other diseases, CVD can be prevented and often only with simple lifestyle modification measures. Although it is generally accepted that all individuals should aim at a healthy lifestyle in order to prevent CVD, there are individuals that are at higher risk of experiencing a severe CVD event such as a heart attack or a stroke. It is thus desirable to effectively identify individuals who are vulnerable for a CVD event and single them out for medical treatment, lifestyle coaching and more healthcare attention.

It is important to note that these higher risk individuals are generally persons who are considered healthy as they show no symptoms. Consequently, the determination of their risk should be easy, non-time consuming and inexpensive. Moreover as they feel healthy, motivating compliance with a treatment or a lifestyle change is an important aim. The results of the health status determination should be presented in a manner that the individual can understand and be motivated to comply with a clinician's recommendation.

The patent and scientific literature demonstrates several methods for determining the risk score of an individual based on multiple parameters. These methods include calculating the Framingham, Reynolds, Rasmunssen, and/or PROCAM risks scores (see, for example, http://www.scopri.ch/riskalgorithms.htm). These risk scores are calculated by a clinician using software or tables. They give an individual a quantitative indication of her/his health status by indicating the likelihood of experiencing an acute event in the future. The calculated risks are used to determine whether an individual falls in the low, medium or high risk categories. Based on the categories certain treatment options can be recommended according to medical guidelines (eg., Circulation 2004, 109, pages 672-693)).

### SUMMARY OF THE INVENTION

It would be advantageous to have a system that allows to plan treatment and/or to recommend lifestyle changes to prevent a disease, e.g. a CVD (cardiovascular disease), by predicting the risk score resulting from the treatment and/or lifestyle changes, respectively. To better address this concern, a first aspect of the invention provides a system for computing a disease risk score, e.g., a CVD (cardiovascular disease) risk score, of a patient based on a recommendation of treatment or lifestyle changes, the system comprising:
an input device for receiving risk parameter values for computing the disease risk score according to a risk score algorithm, and for receiving the recommendation of treatment or lifestyle changes;
an interpreter for computing at least one risk parameter value for computing the disease risk score, on the basis of the recommended treatment or lifestyle changes;
a risk calculator for calculating the disease risk score on the basis of the received risk parameter values and the computed risk parameter value; and
an output device for communicating the calculated disease risk score.

Thanks to the interpreter, the recommended treatment or lifestyle change is "translated" into a value of a risk parameter for computing the disease risk score, thereby allowing calculating the risk score resulting from the treatment or lifestyle change.

In an embodiment of the system, the recommendation of treatment or lifestyle changes is based on information derived from a health record of the patient or a medical rule. The health record of a patient may be a structured record and the system may be arranged to access it. For example, the record may comprise the patient weight. This weight may be used by the system to determine the amounts of dietary supplements for the patient. Alternatively or additionally, the patient record can be a free text record and the system may be arranged to access information comprised in such a free text record using natural language processing (NLP). Optionally, the system may be further arranged to access medical rules in computerized guidelines and for applying a medical rule to make the recommendation of treatment or lifestyle changes.

In an embodiment of the system, the interpreter is arranged for computing the at least one risk parameter value for computing the disease risk score, further based on a compliance level with the recommended treatment or lifestyle changes. This embodiment will allow the user to see the importance of full compliance with the recommended treatment or lifestyle changes, e.g., with her/his daily exercise routine. It may also be helpful in determining a minimum compliance level to see the desired effect.

In an embodiment of the system, the risk parameters values are received from a measurement device. The risk parameters values can be obtained by the system from the measurement device in real time. Alternatively or in addition, the risk parameters values measured by the device may be stored in a data storage and obtained by the system from the data storage when the system requests the data.

In an embodiment, the system further comprises an optimizer for optimizing the calculated risk score based on the recommended treatment or lifestyle changes. The recommended treatment or lifestyle changes may be such that the calculated disease risk score attains a minimum.

In an embodiment, the system further comprises a constrainer for determining constraints for applying to the recommended treatment or lifestyle changes, and wherein the optimizer is arranged for optimizing the calculated risk score based on the recommended treatment or lifestyle changes taking the constraints into account. This can prevent recommending undesired, dangerous, or impossible treatment or lifestyle changes. For example, the length or intensity of physical exercises recommended for a patient who underwent a heart attack must be limited to keep the heart rate below a threshold value. Otherwise the recommendation may lead to grave consequences, even to the patient's death.

In an embodiment of the system, the constraints are determined based on information obtained from a health record of the patient. The system is arranged for analyzing the health record to determine the constraints. Alternatively or additionally, the system may be arranged to alert the user if a recommendation corresponding to the optimized disease risk score is found inconsistent with the patient health record.

In an embodiment of the system, the constraints are determined based on information obtained from a medical rule. The medical rule may be stored in computer-implemented medical guidelines, for example. The system is arranged for analyzing the medical rule to determine the constraints. The rule can provide, for example, the maximum heart rate during physical exercises for a patient who recently underwent a heart attack. Alternatively or additionally, the system may be arranged for identifying and communicating rules relevant to the recommended treatment or lifestyle changes to the user.

In an embodiment of the system, a user is enabled to provide the recommendation of treatment or lifestyle changes. The user may see the effect of various measures of his choice on the calculated disease risk score.

In an embodiment, the system further comprises an analyzer for carrying out a statistical analysis of recommendations provided by a plurality of users. The analyzer may be further arranged to provide the user with the recommendation of treatment or lifestyle changes, based on the statistical analysis of recommendations provided by the plurality of users, e.g., based on the statistical frequency of the recommendation. In an embodiment, the suggestion of lifestyle changes is further based on information derived from a health record of the patient or a depository of medical rules. For example, the statistical frequencies may be binned according to patient characteristics such as age, received treatment, diagnosed conditions, etc.

In an embodiment of the system, the user is a patient or a physician. The system can thus assist the patient in determining preferred treatment and/or lifestyle changes. Alternatively or additionally, the system may guide the physician in determining the most effective treatment or lifestyle changes.

In another aspect, the invention provides a workstation comprising a system set forth.

In another aspect, the invention provides a method of computing a disease risk score, e.g., a CVD (cardiovascular disease) risk score, of a patient based on a recommendation of treatment or lifestyle changes, the method comprising:
an input step for receiving risk parameter values for computing the disease risk score according to a risk score algorithm, and for receiving the recommendation of treatment or lifestyle changes;
a parameterization step for computing at least one risk parameter value for computing the disease risk score, on the basis of the recommended treatment or lifestyle changes;
a risk calculation step for calculating the disease risk score on the basis of the received risk parameter values and the computed risk parameter value; and
a visualization step for communicating the recommendation of treatment or lifestyle changes and/or the calculated disease risk score.

In another aspect, the invention provides a computer program product comprising instructions for causing a processor system to perform a method set forth herein.

It will be appreciated by those skilled in the art that two or more of the above-mentioned embodiments, implementations, and/or aspects of the invention may be combined in any way deemed useful.

Modifications and variations of the workstation, the system, the method, and/or the computer program product, which correspond to the described modifications and variations of the system or the method, can be carried out by a person skilled in the art on the basis of the present description.

### BRIEF DESCRIPTION OF THE DRAWINGS

These and other aspects of the invention are apparent from the above and will be elucidated hereinafter with reference to the drawings.
Fig. 1 is a block diagram of a system for computing a disease risk score of a patient.
Fig. 2 is a flowchart showing steps of a method of computing a disease risk score of a patient.

### DETAILED DESCRIPTION OF EMBODIMENTS

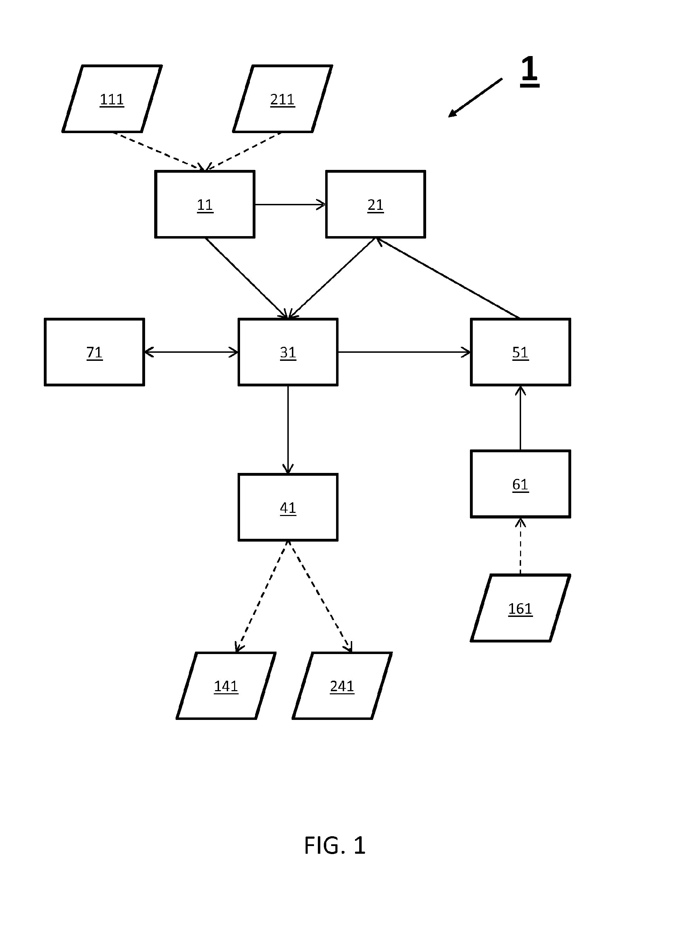

Fig. 1 shows an example of a system 1 for computing a disease risk score of a patient based on a recommendation of treatment or lifestyle changes. The system may be implemented, for example, on a computer system by means of a suitable computer program. The computer system may consist of a workstation. The computer system may also be part of a distributed computer system having a plurality of functional sub-units. The system may comprise a display unit for displaying the images and other information, and a user input device such as a mouse and keyboard. The display and user input device may be integrated into a touch screen.

The system 1 comprises an input device 11 for receiving risk parameter values 111 for computing the disease risk score according to a risk score algorithm. The input device 11 is further arranged for receiving the recommendation 211 of treatment or lifestyle changes. Alternatively, the recommendation 241 computed by the calculator 31 may be optimized by an optimizer 51 and outputted by the output device 41. Optionally, the computed recommendation 241 may take into account some constraints derived by the constrainer 61 from a constraint input 161. The constraint input 161 can be provided by the user of the system 1 or derived from the patient's health record by the constrainer 61. An interpreter 21 is arranged for computing at least one risk parameter value for computing the disease risk score 141, on the basis of the recommended treatment or lifestyle changes. A risk calculator 31 is arranged for calculating the disease risk score on the basis of the received risk parameter values and the computed risk parameter value. The calculated disease risk score is communicated to a user by an output device 41.

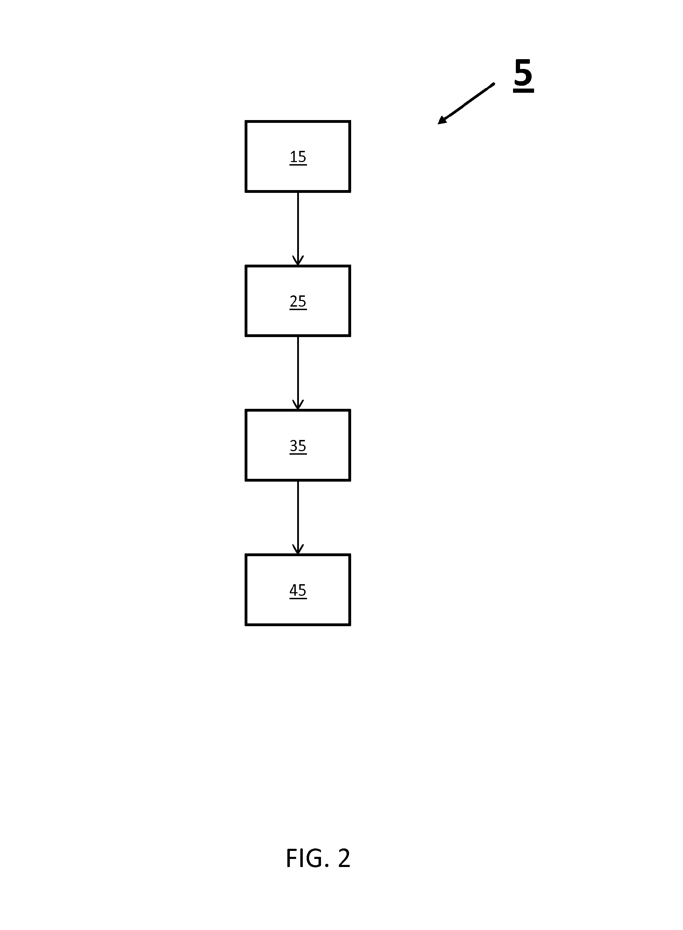

Fig. 2 shows an example of a method 5 of computing a disease risk score of a patient based on a recommendation of treatment or lifestyle changes. The method 5 begins with an input step 15 for receiving the inputs: risk parameter values for computing the disease risk score according to a risk score algorithm and the recommendation of treatment or lifestyle changes. Based on the recommendation of treatment or lifestyle changes, at least one risk parameter value for computing the disease risk score is computed in an parameterization step 25. The received and computed risk parameter values are used at the risk calculation step 35 for calculating the disease score. Finally, the recommended treatment or lifestyle changes and/or the related calculated risk score are communicated in the output step 45.

The embodiments of the system described bellow refer to the cardiovascular disease (CVD). However, a person skilled in the art will have no problem in modifying these embodiments such that they can be applied to other diseases, for example, to leukemia or diabetes.

In an embodiment of the system 1, the recommendation may include a practice of transcendental meditation two times a day for 20 minutes each time. Such practice is known to reduce systolic and diastolic blood pressure by, respectively, 4.7 and 3.2 mm Hg (American Journal of Hypertension, 21, 2008: pages 310-316; 18, 2005: pages 88-98). The interpreter 21 is adapted to use this fact for computing the systolic and diastolic blood pressure values based on the recommendation.

In an embodiment of the system 1, the recommendation may include switching to a Mediterranean style diet. Such a diet can reduce blood triglycerides by 6.14 mg/dL, and increase blood HDL cholesterol by 1.17 mg/dL as well as systolic and diastolic blood pressure by, respectively, 2.35 mm and 1.58 mm (Journal of American College of Cardiology, 57, 2011:1299-1313). The interpreter 21 is adapted to use this fact for computing the systolic and diastolic pressure values and/or the levels of triglycerides and HDL cholesterol, based on the diet recommendation.

In an embodiment of the system 1, the recommendation may include moderate exercises three times per week for the duration of 3 months. With such exercises, the level of blood triglycerides may be reduced by 33 mg/dL in individuals suffering from diabetes or obesity (American Journal of Cardiology, 107, 2011:1168-1172). The interpreter 21 is adapted to use this fact for computing the level of triglycerides in the blood of the patient.

It is to be noted that systolic and diastolic blood pressure and HDL level are important parameters used to calculate the CVD risk score according to Framingham, PROCAM, SCORE and Reynolds algorithms. The triglyceride level is used to calculate the PROCAM risk score.

It is important to note that the calculations assume that the treatment or lifestyle changes will be fully (i.e., 100%) complied with in order to achieve the risk. One can also deal with a case in which the risk is calculated based on only partial (i.e., less than 100%) compliance. Thus, in an embodiment, the interpreter 21 is further arranged for computing at least one risk parameter value for computing the CVD risk score 141, on the basis of the recommended treatment or lifestyle changes and the compliance with the recommended treatment or lifestyle changes expressed, for example, as a fraction of the full compliance. Alternatively, the compliance can be often expressed in absolute numbers, e.g., the number or duration of meditation sessions per day. This feature will allow the user to see the importance of full compliance with the recommended treatment or lifestyle changes, e.g., with her/his daily exercise routine. It may also be helpful in determining a minimum compliance level to see the desired effect.

The recommendation of treatment or lifestyle changes may be based on information derived from a health record of the patient. The health record may include patient's health data such as measurements of body parameters, test results, x-ray or CT (computed tomography) image findings, or responses to health or lifestyle questionnaires. The health record may be a structured health record with formatted data, e.g., a record from a relational database or an unstructured health record, e.g. a free text document or a list of free text documents. An expert system for analyzing patient health records may be employed to obtain the information for the input device 11.

The recommendation of treatment or lifestyle changes may be based on information derived from a medical rule. The medical rule may be comprised in a medical guideline. A computer-implemented medical guideline may be arranged for providing the recommendation to the input device 11. Computer implemented guidelines are described, for example, in a review paper by Gianfranco Damiani et al. entitled The effectiveness of computerized clinical guidelines in the process of care: a systematic review, BMC Health Services Research 2010, 10:2, http://www.biomedcentral.com/1472-6963/10/2. A person skilled in the art will know how to reach other sources with instructions for implementing computerized medical guidelines.

In addition or alternatively to receiving the risk parameter values extracted by an expert system from a health record, the risk parameter values may be measured by and received in real time from measurement devices such as, but not limited to, a heart rate monitor, an EEG (electroencephalograph) activity recorder, and/or a thermometer.

In an embodiment, the system 1 comprises an optimizer 51 arranged for optimizing the CVD risk score. The optimization may be implemented as a search for treatment or lifestyle changes which minimize the CVD risk score. The search may be implemented by an algorithm for traversing a graph which nodes are labeled with candidate treatments or lifestyle changes. Alternatively, the search may be implemented as a random walk or a plurality of random walks on the graph. The candidate treatment or lifestyle changes which correspond to the minimum CVD risk score are the recommended treatment or lifestyle changes. The skilled person will understand that the system 1 may be arranged to optimize the CVD risk score with respect to two or more treatments and/or lifestyle changes, e.g., by recommending a healthy diet, physical exercises and medicines.

In an embodiment, the system 1 further comprises a constrainer 61 for determining constraints for applying to the recommended treatment or lifestyle changes. The constraints can involve the intensity and duration of recommended physical exercises, foods to be consumed, drug dosage, cigarette smoking options. They may result from the patient's characteristics (e.g., age, weight), patient's preferences (e.g., running vs. swimming), comorbidities (e.g., diabetes, clinical depression), social circumstances (the patient lives alone or does not have a car), etc. The optimizer 51 is arranged for optimizing the calculated risk score based on the recommended treatment or lifestyle changes taking the constraints into account. Thus, the optimizer is bound to search only within those candidate treatments and lifestyle changes which satisfy the constraints. Alternatively, the optimizer may be arranged for setting an alarm when the recommended treatment or lifestyle change corresponding to the optimum CVD risk score does not satisfy the constraints.

The constraints may be determined in many ways. In an embodiment of the system 1, they are determined based on information obtained from a health record of the patient. The constrainer 61 is arranged for requesting and receiving the information and for using the information to determine the constraint. For example, the health record may disclose that the patient is allergic to certain drugs or foods and this information can be used by the constrainer 61 to disallow said drugs or foods to be recommended and used for the CVD risk score calculation. Alternatively or additionally, the constraints may be determined based on a medical rule. The medical rule may be requested and obtained by the constrainer 61 from a medical guideline, for example. The rule may define the daily drug dose for different age groups or the duration of exercises necessary to reduce a patient's weight by 5 kg in one month.

In an embodiment of the system 1, a user of the system 1 is enabled to provide the recommendation of treatment or lifestyle changes. Each user's recommendation is stored by the system 1. The system 1 further comprises an analyzer 71 for performing a statistical analysis of the stored recommendations. Optionally, the stored recommendations may be grouped (i.e., binned) based on patient's characteristics such as age, gender, smoker status, blood pressure range, and so on. The analyzer provides the output device with the results of the statistical analysis which are communicated to the user. The results may comprise just the most frequently selected recommendation. Alternatively, the results may include additional statistical information such as results of a test for independence (e.g., Pearsons's chi-square test) of two parameters (e.g., gender and blood pressure).

The users of the system 1 may include patients or medical professionals. Patients may use the system 1 to plan their preferred, personalized lifestyle changes. The system 1 is also capable of raising awareness of patients to the impact of lifestyle changes on their health, therefore increasing patient's motivation to implement these changes. Medical professionals such as physicians may use the system 1 to tailor the recommended treatment to patient's needs, to improve patient's chances for a successful and fast recovery. The system 1 used as a modeling tool can be also useful for educational purposes.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise calls to each other. An embodiment relating to a computer program product comprises computer-executable instructions corresponding to each processing step of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a storage medium, such as a ROM, for example, a CD ROM or a semiconductor ROM, or a magnetic recording medium, for example, a flash drive or a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (1) for computing a disease risk score, e.g., a CVD (cardiovascular disease) risk score, of a patient based on a recommendation of treatment or lifestyle changes, the system comprising:
an input device (11) for receiving risk parameter values for computing the disease risk score according to a risk score algorithm, and for receiving the recommendation of treatment or lifestyle changes;
an interpreter (21) for computing at least one risk parameter value for computing the disease risk score, on the basis of the recommended treatment or lifestyle changes;
a risk calculator (31) for calculating the disease risk score on the basis of the received risk parameter values and the computed risk parameter value; and
an output device (41) for communicating the recommendation of treatment or lifestyle changes and/or the calculated disease risk score.

2. The system according to claim 1, wherein the interpreter (21) is arranged for computing the at least one risk parameter value for computing the disease risk score, further based on a compliance level with the recommended treatment or lifestyle changes.

3. The system according to claim 1, wherein the recommendation of treatment or lifestyle changes is further based on information derived from a health record of the patient or a medical rule.

4. The system according to claim 1, wherein the risk parameters values are received from a measurement device.

5. The system according to claim 1, further comprising an optimizer (51) for optimizing the calculated risk score based on the recommended treatment or lifestyle changes.

6. The system according to claim 5, further comprising a constrainer (61) for determining constraints for applying to the recommended treatment or lifestyle changes, and wherein the optimizer (51) is arranged for optimizing the calculated risk score based on the recommended treatment or lifestyle changes taking the constraints into account.

7. The system according to claim 6, wherein the constraints are determined based on information obtained from a health record of the patient.

8. The system according to claim 6 or 7, wherein the constraints are determined based on information obtained from a medical rule.

9. The system according to claim 1, wherein a user of the system is enabled to provide the recommendation of treatment or lifestyle changes.

10. The system according to claim 9, further comprising an analyzer (71) for carrying out a statistical analysis of recommendations provided by a plurality of users.

11. The system according to claim 10, wherein the analyzer is further arranged to provide the user with the recommendation of treatment or lifestyle changes, based on the statistical analysis of recommendations provided by the plurality of users.

12. The system according to claim 9, wherein the user is a patient or a medical professional.

13. A workstation comprising a system according to claim 1.

14. A method (5) of computing a disease risk score, e.g., a CVD (cardiovascular disease) risk score, of a patient based on a recommendation of treatment or lifestyle changes, the method comprising:
an input step (15) for receiving risk parameter values for computing the disease risk score according to a risk score algorithm, and for receiving the recommendation of treatment or lifestyle changes;
a parameterization step (25) for computing at least one risk parameter value for computing the disease risk score, on the basis of the recommended treatment or lifestyle changes;
a risk calculation step (35) for calculating the disease risk score on the basis of the received risk parameter values and the computed risk parameter value; and
a visualization step (45) for communicating the recommendation of treatment or lifestyle changes and/or the calculated disease risk score.

15. A computer program product comprising instructions for causing a processor system to perform the method according to claim 14.
